# FASCICULE DE BREVET EUROPEEN

(11) **EP 2 959 938 B1**
(45) Date de publication et mention de la délivrance du brevet: **22.03.2017**
(21) Numéro de dépôt: 15171962.2
(22) Date de dépôt: 12.06.2015
(51) Int. Cl.: A61N 1/05

(54) **SONDE IMPLANTABLE COMPRENANT UN MANCHON ENROULABLE AUTOUR D'UN ORGANE TEL QU'UN NERF**
IMPLANTIERBARE SONDE, DIE EINE HÜLSE ZUM UMWICKELN EINES ORGANS, WIE BEISPIELSWEISE EINEN NERV, UMFASST
IMPLANTABLE PROBE COMPRISING A SLEEVE WHICH CAN BE WOUND AROUND AN ORGAN SUCH AS A NERVE

(30) Priorité: 25.06.2014 FR 1455895
(43) Date de publication de la demande: 30.12.2015
(73) Titulaire: Sorin CRM SAS, 92140 Clamart Cedex (FR)
(72) Inventeur: Callegari, Vincent, 1435 Corbais (BE); Mevel, Hervé, 1450 Chastre (St-Géry) (BE); Befahy, Stéphane, 1082 Bruxelles (BE)
(74) Mandataire: Grünecker Patent- und Rechtsanwälte PartG mbB

(56) Documents cités:
- EP-A2- 0 865 800
- DE-A1-102007 036 862
- US-A- 4 602 624
- US-A1- 2009 210 042

## Description

L'invention concerne les "dispositifs médicaux implantables actifs" tels que définis par la directive 90/385/CEE du 20 juin 1990 du Conseil des communautés européennes, plus précisément les implants permettant la stimulation d'un organe de forme cylindrique allongée, et/ou le recueil de potentiels électriques sur un tel organe.

L'invention concerne plus particulièrement la stimulation des nerfs, spécialement la stimulation du nerf vague dans le cas de thérapies dites VNS (*Vagus Nerve Stimulation*).

Toutefois, cette application ne présente aucun caractère limitatif, l'invention pouvant être utilisée pour la stimulation/détection de tout autre organe, ou encore à d'autres fins telles que la délivrance locale d'un agent actif, etc. à cet organe, dès lors que l'organe cible présente une forme cylindrique allongée.

La stimulation du système nerveux est une thérapie reconnue à l'égard de très nombreux troubles tels que l'épilepsie, la douleur, l'insuffisance cardiaque, l'apnée, l'obésité, etc.

Les dispositifs utilisés à cet effet comprennent une sonde pourvue d'une électrode implantée sur le nerf vague et un générateur délivrant des impulsions électriques sur cette électrode.

La thérapie VNS consiste à générer des salves d'impulsions répétitives, synchronisées ou non sur le rythme cardiaque selon le trouble que l'on souhaite traiter, ces impulsions se superposant aux signaux naturellement véhiculés par le système nerveux, organisé en boucle fermée. La stimulation du nerf vague pourra agir en efférence, directement vers un organe, ou en afférence, vers le cerveau pour influer sur le système nerveux central : le signal arbitraire constitué par les impulsions VNS sera alors interprété par le système nerveux central comme une sollicitation que ce dernier va tenter de compenser pour s'y opposer, et empêcher de produire ainsi les effets attendus.

L'invention concerne plus particulièrement le dispositif implanté à l'interface électrode/nerf, qui permet de maintenir les électrodes au contact du nerf ou au voisinage étroit de celui-ci.

Compte tenu de la configuration allongée et approximativement cylindrique d'un nerf périphérique tel que le nerf vague, le dispositif le plus couramment utilisé se présente sous la forme d'un manchon de forme tubulaire, enroulé autour du nerf. Le manchon est généralement réalisé en un élastomère tel que le silicone, du fait de l'excellente biocompatibilité de ce matériau, et il porte sur sa face intérieure, appliquée contre le nerf, les électrodes de stimulation (et/ou de détection).

Un tel manchon est par exemple divulgué par le US 4 602 624 A. Le manchon décrit dans ce document est réalisé à partir de deux feuilles d'élastomère laminées ensemble, l'une d'entre elles ayant été étirée au préalable dans une direction privilégiée de précontrainte. La feuille composite résultante est ensuite découpée pour donner une pièce rectangulaire qui, du fait de la précontrainte de l'une des feuilles, aura naturellement tendance, à l'état libre, à s'enrouler en spirale sur elle-même autour d'un axe perpendiculaire à la direction de précontrainte (une "spirale" étant une courbe plane s'enroulant régulièrement autour d'un point dont elle s'écarte de plus en plus).

Par rapport à un manchon rigide, le manchon décrit dans ce document présente l'avantage d'une simplicité de mise en oeuvre : il suffit au chirurgien de le dérouler, de le passer sous le nerf puis de le relâcher pour qu'il vienne de lui-même s'enrouler autour du nerf. De plus, ce manchon est auto-adaptable : en effet, immédiatement après l'implantation un processus inflammatoire normal produit un gonflement temporaire du nerf, qui disparait ensuite. Si l'on choisit un manchon spiralé souple avec un diamètre intérieur au repos légèrement inférieur au diamètre du nerf, ce manchon - avec ses électrodes - restera toujours étroitement plaqué contre le nerf même si le diamètre de celui-ci varie, et sans risque de compression excessive risquant de détériorer irréversiblement les tissus nerveux.

Ce dispositif n'est cependant pas dépourvu d'inconvénients.

Un *premier inconvénient* se présente au moment de l'implantation : pour poser le manchon, après avoir atteint le nerf cible le chirurgien tire le nerf hors de l'incision qu'il a pratiquée, pour pouvoir glisser le manchon déroulé à l'endroit choisi. Pendant cette manoeuvre, la traction exercée sur le nerf peut entrainer localement, aux extrémités du manchon, des contraintes relativement élevées sur les tissus nerveux, susceptibles d'endommager ces derniers. Une autre cause possible d'endommagement du nerf est la durée de la procédure, qui peut exposer le nerf à l'air pendant trop longtemps. Il est donc nécessaire que la procédure d'implantation du manchon soit très brève, en limitant autant que possible les manipulations du nerf. Également pendant l'implantation, les coins du manchon ont tendance à s'enrouler d'eux-mêmes et gênent l'implantation, ce qui complique la tâche du chirurgien.

Un *deuxième inconvénient,* qui apparait après l'implantation, tient au fait que le bord le plus intérieur du manchon, c'est-à-dire le bord enroulé autour du nerf, vient appuyer contre ce dernier et exerce le long de la ligne de contact une pression ayant tendance à contraindre, voire à déformer, le nerf, avec des effets potentiellement délétères. Le risque existe aussi que lors de l'implantation le chirurgien ait laissé s'enrouler en sens inverse la partie du bord externe du manchon, qui forme alors une seconde spire en sens inverse de la première. Le rayon de courbure n'est alors plus celui qui était prévu, avec pour conséquence un risque de surépaisseur. Enfin, un *troisième inconvénient* est lié au processus de fabrication. Comme indiqué plus haut, le manchon est réalisé en laminant ensemble deux feuilles d'élastomère, avec une précontrainte directionnelle appliquée à l'une d'entre elles. Ces feuilles étant très minces (leur épaisseur typique est d'environ 100 µm), des problèmes d'homogénéité du matériau et de tolérance de l'épaisseur peuvent apparaitre sur l'étendue de la surface d'une même feuille aussi bien qu'entre deux feuilles, ce qui limite la reproductibilité du procédé de production des manchons. Il est certes possible de pallier cet inconvénient en utilisant des feuilles de grande dimension, mais avec une incidence négative sur le processus industriel. Il est également possible d'utiliser des feuilles plus épaisses, mieux contrôlables au cours du processus, mais avec un risque accru d'endommagement du nerf du fait d'une moins grande souplesse et donc d'une moindre aptitude du manchon à se conformer à la morphologie du nerf dans la zone d'implantation.

Un autre type de manchon est décrit dans le DE 10 2007 036 862 A1, qui divulgue un élément en forme de ruban allongé en matériau souple (silicone) portant une ou deux électrodes formées dans une région centrale. Ce ruban est en outre pourvu, d'un côté de la région centrale, d'un trou ou d'une fente transversale, et le côté opposé présente une forme effilée qui, après enroulement autour du nerf, peut être introduite dans la fente de manière à enserrer le manchon sur le nerf, à la manière d'un collier serre-câble emprisonnant un faisceau de fils électriques.

Par rapport au premier type de manchon décrit ci-dessus, ce second type présente des inconvénients multiples :
- il n'est pas "auto-enroulable", en ce sens qu'il ne suffit pas de le dérouler et de relâcher pour qu'il vienne par lui-même se positionner en spirale autour du nerf ; il nécessite au contraire une manipulation du chirurgien pour introduire l'extrémité effilée dans la fente et serrer le manchon autour du nerf ;
- il laisse subsister deux longues parties saillantes s'étendant radialement à partir du site d'implantation (la partie portant la fente, et la partie effilée après introduction dans la fente) ;
- la position des électrodes dans une région centrale du ruban complique le placement précis de ces électrodes au site de stimulation choisi ;
- il n'est pas "auto-adaptable" : le degré de serrage varie avec le diamètre du nerf, qui peut changer par exemple sous l'effet d'un gonflement réactionnel temporaire après implantation ;
- enfin et surtout, le degré de serrage du manchon sur le nerf est totalement opérateur-dépendant puisque ce serrage dépend de la longueur de la partie dépassant de la fente - d'où une possibilité de serrage insuffisant (conduisant à une mauvaise application des électrodes contre le nerf) ou, au contraire, excessif (avec risque de détérioration irréversible des tissus nerveux).

Le besoin subsiste donc d'un manchon auto-enroulable en élastomère mince, pouvant être produit par un procédé industriel performant, avec un degré élevé de reproductibilité, qui n'aurait pu être obtenu jusqu'à présent qu'avec des feuilles épaisses conduisant à des produits non entièrement satisfaisants au plan de leur utilisation.

Le but de l'invention est de résoudre ces problèmes, en proposant une nouvelle structure de manchon auto-enroulable en spirale :
- qui facilite une implantation rapide par le chirurgien, sans introduire de contraintes excessives sur le nerf ;
- qui respecte l'intégrité anatomique du nerf après implantation, tout en continuant d'assurer un maintien satisfaisant du manchon au site d'implantation choisi ; et
- qui puisse être produit par un procédé optimisé du point de vue des contraintes industrielles.

À cet effet, l'invention propose une sonde implantable comprenant un manchon auto-enroulable tel que décrit notamment par le US 4 602 624 A précité, c'est-à-dire comprenant, de manière en elle-même connue, un manchon apte à être enroulé autour d'un organe de forme cylindrique allongée tel qu'un nerf, et comprenant une feuille en matériau élastiquement déformable portant au moins une électrode de détection/stimulation sur une première face de la feuille. La feuille est précontrainte de manière à permettre son auto-enroulement, depuis une position initiale où la feuille est maintenue sous contrainte à l'état déployé, jusqu'à une position finale où la feuille est librement enroulée en spirale en formant un manchon autour de l'organe, avec la première face portant les électrodes tournée vers l'intérieur. La feuille est délimitée par une première largeur définissant un bord extérieur du manchon après enroulement, une seconde largeur opposée définissant un bord intérieur du manchon après enroulement, une première longueur réunissant des premières extrémités homologues de la première et de la seconde largeur, et une seconde longueur opposée réunissant des secondes extrémités homologues de la première et de la seconde largeur.

De façon caractéristique de l'invention, la première largeur est, à ses deux extrémités, raccordée aux deux longueurs par un bord en biseau (c'est-à-dire un bord taillé obliquement au lieu de l'être à angle droit, pour abattre cet angle) respectif formant un angle oblique par rapport à la direction de plus grande dimension de la feuille.

Selon diverses caractéristiques subsidiaires avantageuses :
- le bord en biseau forme par rapport à la direction de plus grande dimension de la feuille un angle compris entre 20 et 45°, et/ou il s'étend sur 15 à 60 % de l'étendue de la feuille dans sa direction de plus grande dimension et/ou sur 20 à 50 % de l'étendue de la feuille perpendiculairement à sa direction de plus grande dimension ;
- la seconde largeur est, à ses deux extrémités, raccordée aux deux longueurs par un autre bord en biseau respectif formant un angle oblique par rapport à la direction de plus grande dimension de la feuille et/ou formant par rapport à la direction de plus grande dimension de la feuille un angle compris entre 30 et 60°, et/ou s'étendant sur 10 à 25 % de l'étendue de la feuille perpendiculairement à sa direction de plus grande dimension ;
- le bord de la feuille dans la région de la première largeur est un bord chanfreiné dans le sens de l'épaisseur, le chanfrein étant orienté vers ladite première face tournée vers l'intérieur ;
- le chanfrein forme par rapport à la direction (Δ) de plus grande dimension de la feuille un angle compris entre 20 et 45°.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 est une vue générale de présentation d'un ensemble de stimulation VNS, montrant le générateur et le nerf vague ainsi que la sonde utilisée.
La Figure 2 est une vue d'un manchon selon l'état de la technique, enroulé autour du nerf vague pendant la procédure d'implantation.
La Figure 3 est une section droite agrandie de l'ensemble formé par le manchon de la Figure 2 enroulé autour du nerf vague, montrant notamment les contraintes que ce manchon peut exercer sur le nerf.
La Figure 4 est une vue en plan du manchon selon l'invention, en configuration déroulée telle que ce manchon se présente au moment de sa fabrication.
La Figure 5 est une section dans le sens de l'épaisseur du manchon de la

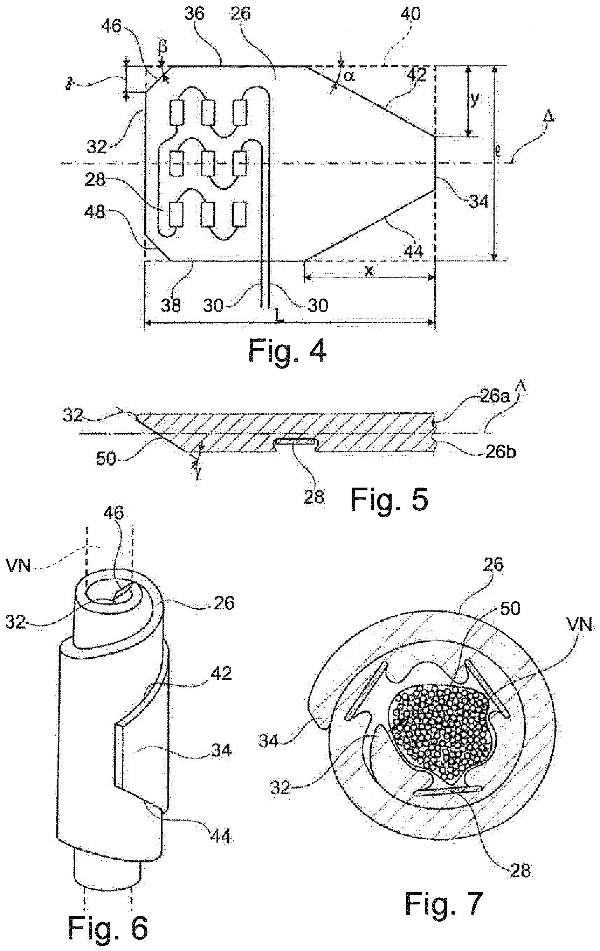

Figure 4, en vue partielle dans la région de l'un des bords, montrant notamment la structure en deux couches superposées laminées ensemble.
La Figure 6 est une vue en perspective du manchon de la Figure 4 dans sa configuration libre, enroulé sur lui-même.

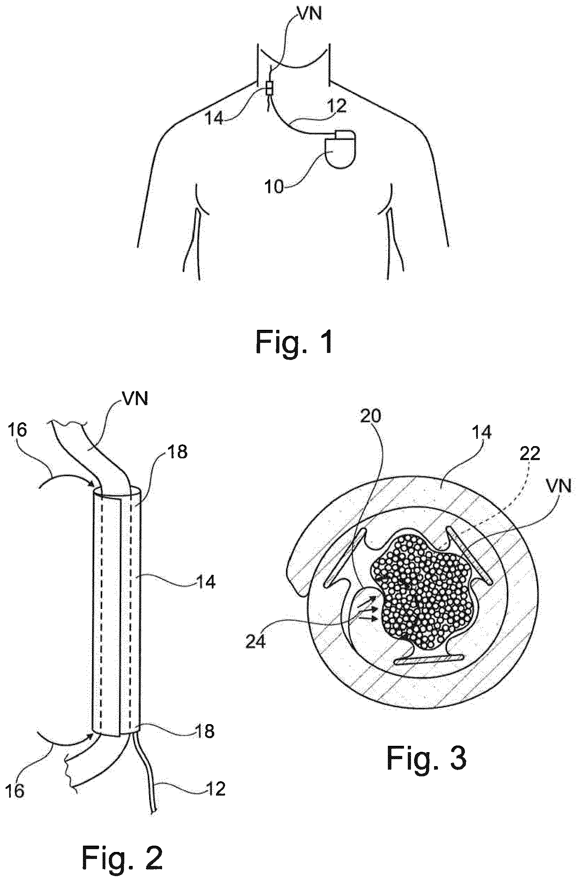

La Figure 7 est une section droite agrandie, homologue de la Figure 3, de l'ensemble formé par le manchon selon l'invention enroulé autour du nerf vague.

On va maintenant décrire un exemple de réalisation de l'invention, sous la forme d'une sonde de stimulation du nerf vague, cet exemple n'étant aucunement limitatif comme on l'a indiqué en introduction.

Sur la Figure 1, la référence 10 désigne le boitier d'un générateur implantable de stimulation VNS. Les impulsions de stimulation générées sont délivrées par une sonde portant à sa partie distale un manchon 14 pourvu d'électrodes appliquées contre le nerf vague VN et susceptible de stimuler celui-ci par les salves d'impulsions produites par le générateur 10.

La Figure 2 est une vue d'un manchon 14 selon l'état de la technique, enroulé autour du nerf vague VN pendant la procédure d'implantation.

Pour poser le manchon, le chirurgien a dû tirer le nerf vague VN hors de l'incision qu'il avait pratiquée, pour pouvoir y glisser le manchon déroulé à l'endroit choisi. Après auto-enroulement en spirale, le manchon 14 se présente comme illustré sur la Figure 2. Dans cette configuration particulière, des contraintes sont exercées par le manchon cylindrique dans la région 16 du fait de la discontinuité de rigidité entre la partie du nerf enfermée à l'intérieur du manchon 14, et la partie libre au-delà du manchon. Cette discontinuité entre une partie où le nerf est immobilisé et celle où il est libre crée localement des contraintes à l'endroit de la transition, contraintes qui peuvent endommager les tissus nerveux.

Un autre inconvénient, également propre aux manchons de l'art antérieur, tient au fait que si l'on considère l'extrémité la plus intérieure du manchon 20 enroulé en spirale, le fait que cette extrémité soit enserrée par le reste du manchon a pour conséquence d'exercer des contraintes sur le nerf VN dans la région 22 située au voisinage de cette extrémité (contraintes schématisées par les flèches 24), qui ont pour effet de déformer le nerf de façon permanente, avec des effets potentiellement délétères.

Ces différents inconvénients, ainsi que ceux exposés en introduction, peuvent être résolus par un manchon réalisé selon les enseignements de l'invention, illustré aux Figures 5 à 7.

La Figure 4 est une vue en plan du manchon selon l'invention, en configuration déroulée telle que ce manchon se présente au moment de sa fabrication.

Le manchon 26 selon l'invention est réalisé à partir de deux feuilles d'élastomère 26a et 26b (Figure 5) laminées ensemble, par exemple de silicone, l'une des feuilles ayant été soumise au préalable à une précontrainte d'étirement dans la direction Δ, qui est dans cet exemple la direction de plus grande dimension de la feuille 26. Comme expliqué dans le US 4 602 624 A précité, cette technique permet de rendre le manchon auto-enroulable en spirale lorsque la feuille 26, après fabrication du manchon, ne sera plus soumise à aucune contrainte extérieure, conduisant à la configuration enroulée illustrée Figure 6.

Le silicone est choisi préférentiellement comme matériau de base pour le manchon implantable en raison de ses excellentes propriétés de biocompatibilité, tant au niveau de la biotolérance (l'implant n'induit pas de dommage à l'hôte : absence de toxicité et d'endommagement mécanique des tissus) que de sa biostabilité (l'implant résiste aux conditions induites par l'hôte).

La feuille 26 porte, dans la région destinée à venir en contact avec le nerf vague après enroulement (la région située à gauche sur la Figure 4) un certain nombre d'électrodes 28 rapportées en surface de la feuille ou enfoncées dans l'épaisseur du matériau élastomère. Ces électrodes 28 sont reliées à des fils 30 destinés à être reliés au générateur d'impulsions 10. Dans l'exemple illustré sur la Figure 4, ces électrodes 28 sont distribuées de manière uniforme le long de l'axe d'enroulement du manchon 26 et elles sont reliées entre elles de manière à constituer une matrice de contacts de type quasi-tripôle (anode/cathode/anode ou l'inverse) reliés aux microcâbles 30 correspondants.

Le manchon est réalisé à partir de la feuille 26 qui présente une forme rectangulaire, avec un côté 32 formant une première largeur (qui viendra à l'intérieur de la spirale après enroulement du manchon), un deuxième côté 34 opposé formant une seconde largeur (qui viendra à l'extérieur du manchon après enroulement), et deux côtés 36, 38 formant une première et une seconde longueur reliant entre elles les côtés des largeurs 32, 34. De façon caractéristique de l'invention, les coins à angle droit de cette feuille rectangulaire sont découpés (par estampage, découpe à la lame ou tout autre processus industriel adapté) de manière à éliminer les régions délimitées par les tiretés 40, formant ainsi des bords en biseau 42, 44, 46, 48. Dans la région située du côté 34, les bords en biseau 42 et 44 forment avec les longueurs 36, 38 (elles-mêmes parallèles à l'axe Δ) un angle *α* de 20° à 45° par exemple, de sorte que l'étendue de la partie 40 éliminée pour former le biseau s'étend sur une longueur *x* de l'ordre de 15 à 60 % de la longueur totale *L* de la feuille 26, et sur une largeur *y* de l'ordre de 20 à 50 % de la largeur totale ℓ de la feuille 26. Quant aux biseaux 46, 48 du côté opposé, ceux-ci forment un angle *β* de 30° à 60° et s'étendent sur une largeur z de 10 à 25 % de la largeur totale ℓ de la feuille 26.

Par ailleurs, dans le sens de l'épaisseur, l'extrémité 32 forme un bord chanfreiné 50, le chanfrein étant tourné vers la face de la feuille qui sera appliquée contre le nerf (c'est-à-dire la face portant les électrodes 28). Ce chanfrein est incliné d'un angle *γ* compris entre 20° et 45° par exemple. Avec la configuration que l'on vient de décrire, dans sa configuration enroulée le manchon selon l'invention prend la forme illustrée Figure 6, avec un aspect ressemblant à un croissant de viennoiserie (de forme droite), du fait des bords en biseau 42, 44, qui donnent à la partie enroulée côté extérieur une forme de languette.

Cette languette facilite les opérations d'implantation, dans la mesure où le manchon peut être manipulé sans risque de replier des coins du manchon vers l'intérieur, ce qui formerait des surépaisseurs dommageables.

De plus, la forme en "croissant" rend immédiatement visible un enroulement inversé par mégarde, c'est-à-dire où la région portant les électrodes (à gauche sur la Figure 4) viendrait recouvrir la région opposée (la région droite sur la Figure 4), au lieu que ce soit l'inverse. Comme les biseaux 46, 48 sont de beaucoup plus petite dimension que les biseaux 42, 44, la forme typique avec languette comme sur la Figure 6 serait absente, révélant immédiatement l'enroulement à l'envers.

Un autre avantage de cette forme en croissant réside dans le gradient de rigidité du manchon en configuration enroulée, la rigidité diminuant progressivement depuis le centre du manchon jusqu'aux extrémités. La plus grande souplesse aux extrémités permet d'exercer localement moins de contraintes sur le nerf (à la différence des manchons de la technique antérieure, comme illustré Figure 2), tandis que dans la région centrale le serrage exercé par le manchon est maximal, ce qui permet de bien maintenir les électrodes en appui contre le nerf.

Pour réaliser le manchon de l'invention, il est possible d'utiliser des feuilles d'épaisseur relativement mince (de l'ordre de 100 µm), ce qui conduit à des manchons très souples, et donc très bien tolérés, sans compromis sur la facilité de pose et avec une transition très progressive entre le nerf et le manchon.

En ce qui concerne le chanfrein 50, l'avantage procuré par celui-ci est illustré sur la Figure 7, à comparer avec la Figure 3, qui est une représentation semblable pour un manchon conventionnel : dans le cas de l'invention, l'épaisseur de matière est moindre dans la région du bord 32 en contact avec le nerf, de sorte que celui-ci est soumis à beaucoup moins de contraintes, éliminant ainsi les risques de déformation, voire d'écrasement, subis localement par le nerf.

## Revendications

1. Une sonde implantable comprenant un manchon apte à être enroulé autour d'un organe de forme cylindrique allongée tel qu'un nerf (VN), ce manchon comprenant une feuille (26) en matériau élastiquement déformable portant au moins une électrode (28) de détection/stimulation sur une première face de la feuille,
la feuille (26) étant précontrainte de manière à permettre son auto-enroulement depuis une position initiale où la feuille est maintenue sous contrainte à l'état déployé jusqu'à une position finale où la feuille est librement enroulée en spirale en formant un manchon autour de l'organe, avec la première face portant les électrodes tournée vers l'intérieur,
la feuille (26) étant délimitée par une première largeur (34) définissant un bord extérieur du manchon après enroulement, une seconde largeur (32) opposée définissant un bord intérieur du manchon après enroulement, une première longueur (36) réunissant des premières extrémités homologues de la première et de la seconde largeur, et une seconde longueur (38) opposée réunissant des secondes extrémités homologues de la première et de la seconde largeur,
**caractérisée en ce que** la première largeur (34) est, à ses deux extrémités, raccordée aux deux longueurs (36, 38) par un bord en biseau respectif formant un angle oblique (*α*) par rapport à la direction (Δ) de plus grande dimension de la feuille.

2. La sonde de la revendication 1, dans laquelle le bord en biseau (42, 44) forme par rapport à la direction (Δ) de plus grande dimension de la feuille un angle (*α*) compris entre 20 et 45°.

3. La sonde de la revendication 1, dans laquelle le bord en biseau (42, 44) s'étend sur 15 à 60 % de l'étendue (*L*) de la feuille dans sa direction (Δ) de plus grande dimension.

4. La sonde de la revendication 1, dans laquelle le bord en biseau (42, 44) s'étend sur 20 à 50 % de l'étendue (ℓ) de la feuille perpendiculairement à sa direction (Δ) de plus grande dimension.

5. La sonde de la revendication 1, dans laquelle la seconde largeur (32) est, à ses deux extrémités, raccordée aux deux longueurs (36, 38) par un autre bord en biseau respectif (46, 48) formant un angle oblique (*β*) par rapport à la direction (Δ) de plus grande dimension de la feuille.

6. La sonde de la revendication 5, dans laquelle l'autre bord en biseau (46, 48) forme par rapport à la direction (Δ) de plus grande dimension de la feuille un angle (*β*) compris entre 30 et 60°.

7. La sonde de la revendication 5, dans laquelle l'autre bord en biseau (46, 48) s'étend sur 10 à 25 % de l'étendue (ℓ) de la feuille perpendiculairement à sa direction (Δ) de plus grande dimension.

8. La sonde de la revendication 1, dans laquelle le bord de la feuille dans la région de la première largeur (32) est un bord chanfreiné dans le sens de l'épaisseur, le chanfrein (50) étant orienté vers ladite première face tournée vers l'intérieur.

9. La sonde de la revendication 8, dans laquelle le chanfrein (50) forme par rapport à la direction (Δ) de plus grande dimension de la feuille un angle (*γ*) compris entre 20 et 45°.

## Patentansprüche

1. Implantierbare Sonde umfassend eine Hülse, die dazu eingerichtet ist, um ein zylinderförmiges längliches Organ wie einen Nerv (VN) gewickelt zu werden, wobei diese Hülse eine Folie (26) aus einem elastisch verformbaren Material umfasst, die auf einer ersten Fläche der Folie mindestens eine Erfassungs-/Stimulationselektrode (28) trägt,
wobei die Folie (26) derart vorgespannt ist, dass eine Selbstwicklung aus einer Ursprungsstellung, in der die Folie unter Vorspannung im ausgebreiteten Zustand gehalten wird, in eine Endstellung, in der die Folie unter Ausbildung einer Hülse um das Organ spiralförmig frei aufgewickelt ist, ermöglicht wird, wobei die die Elektroden tragende erste Fläche nach innen gerichtet ist,
wobei die Folie (26) durch eine erste Breite (34), die nach der Aufwicklung einen äußeren Rand der Hülse bestimmt, eine gegenüberliegende zweite Breite (32), die nach der Aufwicklung einen inneren Rand der Hülse bestimmt, eine erste Länge (36), die erste homologe Enden der ersten und der zweiten Breite vereint, und eine gegenüberliegende zweite Länge (38), die zweite homologe Enden der ersten und der zweiten Breite vereint, begrenzt ist,
**dadurch gekennzeichnet, dass** die erste Breite (34) an beiden Enden durch einen jeweiligen abgeschrägten Rand, der einen schrägen Winkel (α) zur Richtung (Δ) der größten Abmessung der Folie bildet, mit den zwei Längen (36, 38) verbunden ist.

2. Sonde nach Anspruch 1, in der der abgeschrägte Rand (42, 44) einen Winkel (α) von 20 bis 45° zur Richtung (Δ) der größten Abmessung der Folie bildet.

3. Sonde nach Anspruch 1, in der der abgeschrägte Rand (42, 44) sich über 15 bis 60% der Erstreckung (*L*) der Folie in Richtung (Δ) ihrer größten Abmessung erstreckt.

4. Sonde nach Anspruch 1, in der der abgeschrägte Rand (42, 44) sich über 20 bis 50% der Erstreckung (*l*) der Folie senkrecht zur Richtung (Δ) ihrer größten Abmessung erstreckt.

5. Sonde nach Anspruch 1, in der die zweite Breite (32) an ihren beiden Enden durch einen weiteren jeweiligen abgeschrägten Rand (46, 48), der einen schrägen Winkel (β) zur Richtung (Δ) der größten Abmessung der Folie bildet, mit beiden Längen (36, 38) verbunden ist.

6. Sonde nach Anspruch 5, in der der weitere abgeschrägte Rand (46, 48) einen Winkel (β) von 30 bis 60° zur Richtung (Δ) der größten Abmessung der Folie bildet.

7. Sonde nach Anspruch 5, in der der weitere abgeschrägte Rand (46, 48) sich über 10 bis 25% der Erstreckung (*l*) der Folie senkrecht zur Richtung (Δ) ihrer größten Abmessung erstreckt.

8. Sonde nach Anspruch 1, in der der Rand der Folie im Bereich der ersten Breite (32) ein in Richtung der Dicke abgefaster Rand ist, wobei die Fase (50) zur nach innen gerichteten ersten Fläche hin gerichtet ist.

9. Sonde nach Anspruch 8, in der die Fase (50) einen Winkel (γ) zwischen 20 und 45° zur Richtung (Δ) der größten Abmessung der Folie bildet.

## Claims

1. An implantable lead comprising a cuff adapted to be wound around an elongate cylindrical organ such as a nerve (VN), the cuff comprising a sheet (26) of elastically deformable material having at least one detection/stimulation electrode (28) on a first face of the sheet;
the sheet (26) being pre-stressed such that it is self-winding from an initial position, in which the sheet is held under stress in the deployed state, to a final position, in which the sheet is loosely spirally wound to form a cuff around the organ, with the first face carrying the electrodes facing inwards,
the sheet (26) being delimited by a first width (34) defining an external edge of the cuff after it has been wound, a second opposite width (32) defining an internal edge of the cuff after it has been wound, a first length (36) joining first homologous ends of the first and the second width, and a second opposite length (38) joining second homologous ends of the first and the second widths, **characterized in that** the first width (34) is connected at both ends to the two lengths (36, 38) by a respective bevel edge forming an oblique angle (α) with respect to the direction (Δ) of greatest dimension of the sheet.

2. The lead of claim 1, wherein the beveled edge (42, 44) forms an angle angle (α) between 20 and 45°, relative to the direction (Δ) of largest dimension of the sheet.

3. The lead of claim 1, wherein the beveled edge (42, 44) extends across 15 to 60% of the extent (*L*) of the sheet in the direction (Δ) of greatest dimension.

4. The lead of claim 1, wherein the beveled edge (42, 44) extends across 20 to 50% of the extent (*l*) of the sheet perpendicular to the direction (Δ) of greatest dimension.

5. The lead of claim 1, wherein the second width (32) is connected at both ends to the two lengths (36, 38) by another respective bevel (46, 48) forming an oblique angle (*β*) relative to the direction (Δ) of greatest dimension of the sheet.

6. The lead of claim 5, wherein the other beveled edge (46, 48) forms an angle (*β*) between 30 and 60°, relative to the direction (Δ) of greatest dimension of the sheet.

7. The lead of claim 5, wherein the other beveled edge (46, 48) extends across 10 to 25% of the extent (*l*) of the sheet perpendicular to the direction (Δ) of greatest dimension.

8. The lead of claim 1, wherein the edge of the sheet in the first width region (32) is a chamfered edge in the thickness direction, the chamfer (50) being oriented toward the first face facing inwardly.

9. The lead of claim 8, wherein the chamfer (50) forms an angle (*γ*) between 20 and 45° with respect to the direction (Δ) of greatest dimension of the sheet.
